(19) Europäisches Patentamt
European Patent Office
Office européen des brevets

(11) Publication number: **0 108 822**
**B1**

(12) **EUROPEAN PATENT SPECIFICATION**

(45) Date of publication of patent specification: **13.08.86**

(51) Int. Cl.[4]: **C 07 C 93/26,** A 61 K 31/135, A 61 K 31/22

(21) Application number: **82110433.8**

(22) Date of filing: **11.11.82**

# (54) Antiglaucoma compounds.

(43) Date of publication of application:
**23.05.84 Bulletin 84/21**

(45) Publication of the grant of the patent:
**13.08.86 Bulletin 86/33**

(84) Designated Contracting States:
**BE DE FR GB SE**

(56) References cited:
**EP-A-0 067 910**
**WO-A-82/03327**
**US-A-4 138 581**
**US-A-4 275 074**

(73) Proprietor: **USV PHARMACEUTICAL CORPORATION**
**1 Scarsdale Road**
**Tuckahoe New York (US)**

(72) Inventor: **Loev, Bernard**
**42 Penny Lane**
**Scarsdale New York (US)**
Inventor: **Lee, Daw-Yuan**
**54 Vernon Drive**
**Scarsdale New York (US)**
Inventor: **Youssefyeh, Raymond**
**435 Mertling Avenue**
**Tarrytown New York (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath**
**Maximilianstrasse 58**
**D-8000 München 22 (DE)**

**EP 0 108 822 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to compounds of formula I

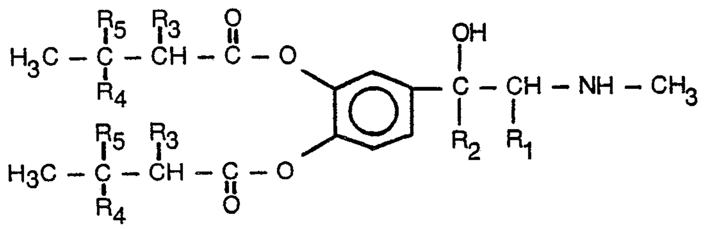

(I)

wherein each of $R_1$, $R_2$ and $R_3$ is independently hydrogen, methyl or ethyl, and each of $R_4$ and $R_5$ is independently methyl or ethyl, and their pharmaceutically acceptable acid addition salts.

It is to be understood that many of the above-identified compounds have one or more asymmetric carbon atoms and that the present invention includes all stereoisomers of the new compounds.

The compounds of formula I and their acid addition salts are suitable for the treatment of glaucoma.

The use of epinephrine and its salts and derivatives in the treatment of glaucoma is known in the art (F. E. Leaders, et al. Arch. int. Pharmacodyn. *183,* 93—106 (1970)). US—A—3,809,714 discloses the di-pivaloyl ester of epinephrine as having anti-glaucoma activity. The 3,3-dimethyl-butyric acid diester of N-t-butyl-α-methyl-norepinephrine having the structure

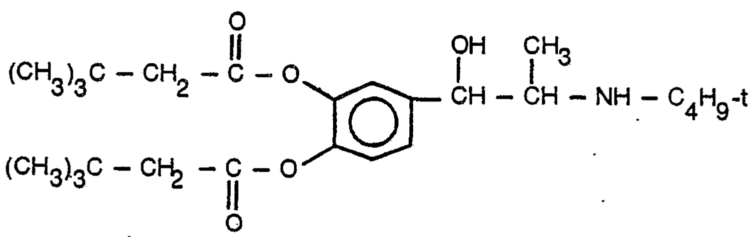

has been reported in GB—A—1,298,771 to show bronchodilator activity.

US—A—4138581 discloses esters of 3,4-dihydroxy-α-(amino- and n-substituted amino methyl) benzyl alcohols and esters of 3-hydoxymethyl-4-hydroxy-α-(amino- and n-substituted amino-methyl)benzyl alcohols which produce useful sympathomimetic effects, for example bronchodilation, of long duration in warm-blooded mammals. Among other compounds the preparation of 3,4-bis (3,3-dimethylbutanoyloxy)-α-(tert.-butylaminomethyl)benzyl alcohol hydrochloride and 3,4-bis (2,2-dimethylpentanoyloxy)-α-(methylaminomethyl)benzyl alcohol methanesulfonate is disclosed.

For these known compounds which are reported to have anti-glaucoma activity, the activity is weak at low concentrations and is of relatively short duration at such concentrations.

Some antiglaucoma drugs tend to give an initial increase in the intraocular pressure after administration. While this increase is usually fleeting, it is an undesirable effect for individuals having glaucoma.

Other undesirable effects of known anti-glaucoma drugs are mydriasis — the dilation of the pupil — and contralateral activity, i.e., the reduction of the intraocular pressure of the eye which is not being treated with the drug. This is an indication of a systemic effect which is usually accompanied by side effects such as tachycardia.

It is the object of the present invention to provide new compounds useful for the treatment of glaucoma.

Said object is achieved by compounds according to claim 1.

It has been found that the topical administration to a glaucomatous eye of a substituted alkyl diester of epinephrine of formula 1 or an alkyl derivative thereof, preferably as a solution of a pharmaceutically acceptable, non-toxic acid addition salt thereof, will result in a significant reduction, i.e., of the order to about 5 to 7 mm., in the intraocular pressure of the glaucomatous eye for a period of about 24 hours.

This reduction in the intraocular pressure over the 24 hour period occurs at concentrations of about 0.05 to 0.10 percent by weight. At similar concentrations the dipivalyl ester of epinephrine provides lower reductions over a period of only 5 hours; while the 3,3 dimethylbutyric acid di-ester of N-t-butyl-α-methyl-norepinephrine provides still lower reductions for a period of only 3.5 hours.

The novel esters of the present invention exhibit activity at concentrations as low as 0.005 and 0.001 percent. The above-mentioned known esters exhibit no activity at these concentrations.

The preferred compounds of the present invention are those wherein the acid addition salt is the hydrochloride or the prechlorate, $R_1$ is hydrogen or methyl, $R_2$ and $R_3$ are hydrogen and $R_4$ and $R_5$ are methyl.

Further, a compound wherein $R_1$, $R_3$, $R_4$ and $R_5$ are methyl and $R_2$ is hydrogen, a compound wherein $R_1$, $R_2$ and $R_3$ are hydrogen, $R_4$ is methyl and $R_5$ is ethyl and a compound wherein $R_1$, $R_2$, $R_4$ and $R_5$ are methyl and $R_3$ is hydrogen is preferred.

The new products of the present invention are prepared by reduction of the corresponding keto compound of formula II

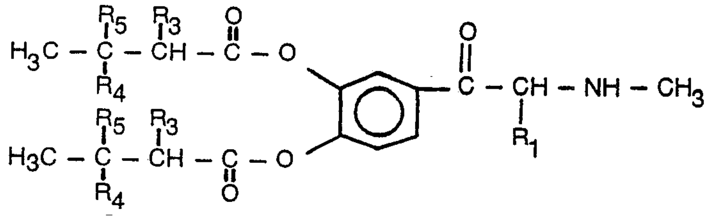

(II)

to produce compounds in which $R_2$ is hydrogen. Alternatively, compounds in which $R_2$ is methyl or ethyl can be produced by reaction of the keto-carbonyl group of the aforsaid keto compounds to form the requisite secondary alcohol group. This is accomplished by reaction of methyl or ethyl magnesium halide or equivalent reagents known to react with carbonyl groups to form secondary alcohols.

If desired, the present new compounds can also be prepared by acylating compounds of formula III

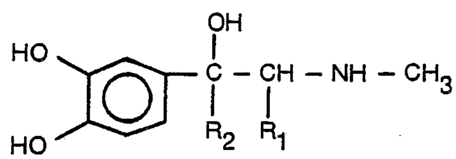

(III)

with an acylating derivative of an acid of formula IV

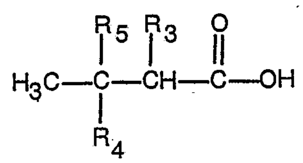

(IV)

Said products are optionally connected to a pharmaceutically acceptabnle acid salt.

The aforesaid reduction process, i.e. reduction of a carbonyl group to a secondary alcohol group, can be accomplished by any of the methods commonly employed for such conversions including chemical reduction, e.g. active metals and mineral acids such as zinc or tin and hydrochloric acid, or using catalytic reduction, e.g. hydrogen gas over metal catalysts such as palladium and nickel. If desired, metal hydrides such as metal aluminum hydrides and borohydrides can also be used. Usually, for commercial production, catalytic reduction is preferred particularly employing several atmospheres of pressure of hydrogen gas over noble metal catalysts such as palladium or platinum, usually on a catalyst such as carbon.

Reaction of the carbonyl compound with Grignard reagents, e.g. ethyl magnesium chloride and methyl magnesium bromide, is accomplished using known methods. The Grignard reagent need merely to be contacted with the carbonyl compound for reaction to occur.

The aforesaid reactions are conveniently carried out in the presence of a solvent for the reactants. Such solvents include, for example, ester solvents such as ethyl acetate and butyl acetate, N,N-dialkyl amides such as dimethyl formamide and diethyl acetamide, acetonitrile, tetrahydrofuran, dioxane, acetic acid, methylene chloride, ethylene chloride, and similar such solvents.

The aforesaid acylation reaction can be carried out using known derivatives of the said acids, including for example acyl halides, anhydrides, mixed anhydrides, lower alkyl esters, and the free acids in the presence of for example, carbodiimides and carbonyl diimidazoles.

Said reactions will occur at room temperature and even lower to about 0°C. The use of elevated temperatures is for convenience in that it permits somewhat shortened reaction periods. Temperatures ranging from 0°C up to the reflux temperature of the reaction system can be used.

Where hydrogen halide is involved as a reaction product, it is convenient to use hydrogen halide acceptors, e.g. tertiary organic amines e.g. trimethylamine, pyridine or picolines.

The present new compounds are also preparable by the reaction of the corresponding epoxide of the formula V

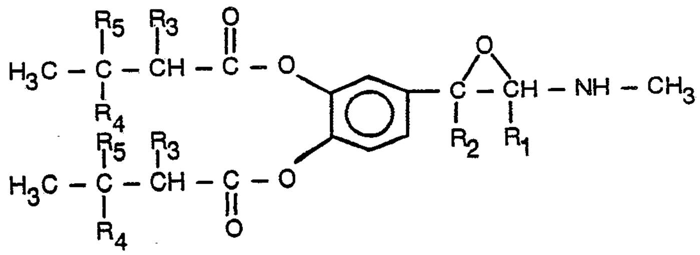

(V)

with an amine $CH_3NH_2$ to form the hydroxyl amine under known reaction conditions, which preferably utilize a solvent as described hereinbefore.

The invention will be illustrated in the examples which follow.

## Example I

A. α-Methylamino-3,4-bis(3,3-dimethylbutanoyloxy)-acetophenone perchlorate

To a suspension of 6.6 g (30 mM) of α-methylamino-3,4-dihydroxy-acetophenone hydrochloride in ethylacetate (75 ml) was added 30 mM of perchloric acid as a 70% aqueous solution with stirring. The solution was warmed to reflux for one hour. Then 83.3 ml of 3,3-dimethylbutanoyl chloride was added. After two hours at reflux, the mixture was cooled to room temperature, and the precipitate was filtered with the assistance of ether to give the product as on off-white colored solid. The product can be purified by recrystallization with a mixture of ethyl acetate and methanol (m.p. 190—192°C). The starting material was obtained by the method of Hussain and Truelove, J. Pharm. Sci. *65,* 1510 (1976).

B. 2-Methylaminomethyl-1[3,4-bis(3,3-dimethylbutanoyloxyphenyl]-1-ethanol perchlorate

To a solution of α-methylamino-3,4-bis (3,3-dimethylbutanoyloxy)-acetophenone perchlorate (14g, 30mM) in alcohol (300 ml) was added 0.5 g of Adams catalyst (platinum oxide). The mixture was hydrogenated in a Parr shaker for two hours. After filtration to remove the catalyst, the solvent was evaporated on a rotavapor, then was pumped to dryness. The oil thus obtained was dissolved in ether (50ml) and scratched to give a precipitate. After filtration the product was obtained as a white solid, m.p. 118—120°C.

C. 2-Methylamino-1-[3,4-bis(3,3-dimethylbutanoyloxy) phenyl)]-ethanol hydrochloride

The methanol solution of the perchlorate from *B* (2.4 g) was passed through an ion-exchange column (IRA-400, 200 g) using methanol as eluate. The eluate collected was evaporated in a rotavapor to dryness. The solid residue was recrystallized from a mixture of ethyl acetate and ether to give the product as a white solid, m.p. 136—138°C.

## Example II

A. α-Chloro-3,4-bis(3,3-dimethylbutanoyloxy)-acetophenone

To a suspension of α-chloro-3,4-dihydroxyacetophenone (9.4 g, 50 mM) in ethyl acetate (50 ml) was added 10 mM of perchloric acid as a 70% aqueous solution with stirring. The mixture was warmed to reflux while 3,3-dimethylbutanoyl chloride (60 ml) was added. After stirring for 30 minutes, it was cooled to room temperature. The solution was quickly washed with water once, and the organic layer, without drying, was immediately evaporated on a rotavapor. The oil thus obtained was dissolved in chloroform and filtered through a short silica gel "dry-column" to remove the dark material. The eluate collected was evaporated to an oil which upon treatment with hexane and scratching gave the product as an off-white colored solid (9.2 g), m.p. 49—51°C.

B. 2-Methylamino-1-[3,4-bis(3,3-dimethylbutanoyloxy)phenyl]-ethanol hydrochloride

To a solution of 11.5 g (0.03 ml) af a α-chloro-3,4-bis (3,3-dimethylbutanoyloxy)-acetophenone in 100 ml ethanol was added a solution of 8.5 g (0.07 mol) of methylbenzylamine in 100 ml ethanol. The mixture was stirred for about 15 hours at room temperature. After removal of the solvent the residue was diluted with water and extracted with ether. The ether extract was washed with water and dried over anhyd. $MgSo_4$. The ether solution was then evaporated and the residue dissolved in ethanol and hydrogenated over a 5% Pd-C catalyst. After removal of the catalyst by filtration the solution was treated with anhydrous hydrogen chloride and evaporated to yield a white solid, m.p. 136—138°C.

## Example III

A. 3,4 Bis(3,3-dimethylbutanoyloxy) propiophenone

To a solution of 39 g (0.23 moles) of 3,4-dihydroxypropiophenone in 400 ml of pyridine at 60°C was slowly added 65 g (67 ml, 0.48 moles) of 3,3-dimethylbutanoyl chloride. The mixture was stirred at 100° for two hours. It was then allowed to cool and was filtered and washed with ether. The filtrate was evaporated to dryness. The oily residue was taken up in ether; washed with water, 3% HCl solution and again with

water. It was then dried over anhyd. $MGSO_4$ and evaporated to dryness giving the oily product which crystallized on standing overnight.

B. α-Bromo-3,4-bis(3,3-dimethylbutanoyloxy)-propiophenone

To a mixture of 74 g (0.17 mole) of the crude 3,4-bis(3,3-dimethylbutanoyloxy) propiophenone and 25 g (9.25 mole) of calcium carbonate in 500 ml of dichloromethane was slowly added a solution of 35 g (11 ml, 0.21 mole) of bromine in 50 ml of dichloromethane. The reaction mixture was stirred and heated to 40°C until evolution of hydrogen bromide ceased. Nitrogen was passed through the solution to remove the remaining hydrogen bromide. Removal of the solvent to dryness gave a crude oil product.

C. α-Benzylmethylamino-3,4-bis(3,3-dimethylbutanoyloxy) propiophenone

A mixture of 11.1 g (0.025 mole) of α-bromo-3,4-bis(3,3-dimethylbutanoyloxy) propiophenone and 5 g potassium iodide in 120 ml dimethylformamide was stirred at room temperatures for 15 minutes. A solution of 6.5 ml of benzyl methylamine in 10 ml of dimethylformamide was then slowly added and stirred continued for 16 hours. It was poured on ice-water, extracted with hexane, washed with water, dried over magnesium sulfate and evaporated to dryness. The remaining oily residue was crystallized from methanol, m.p. 59—61°C.

D. 1-[3,4-Bis(3,3-dimethylbutanoyloxy)phenyl]-2-methylamino-1-propanol hydrochloride

A mixture of 2.7 g (5.6 mmoles) of α-benzylmethylamino-3,4-bis (3,3-dimethylbutanoyloxy) propiophenone and 300 mg of 5% Pd/C in 200 ml ethanol was hydrogenated overnight. After filtration and removal of the solvent the oily residue was crystallized from hexane, m.p. 86—8°C. The above base, dissolved in 10 ml of methanolic hydrogen chloride, was stirred for 15 minutes. The solvent was removed and the residue was twice washed with ether and decanted. The remaining oily product was crystallized from ethyl acetate-ether, m.p. 166—7°C.

Example IV

In the same way as described in Example III except using 2,3,3-trimethylbutanoyl chloride as the starting material there was obtained 1-[3,4-bis (2,3,3-trimethylbutanoyloxy)phenyl]-2-methylamino-1-propanol hydrochloride.

Example V

In the same way as described in Example II except using 3,3-dimethylpentanoyl chloride as the starting material, there was obtained 2-methylamino-1-[3,4-bis (3,3-dimethylpentanoyloxy)phenyl]-ethanol hydrochloride.

Example VI

A. α-Benzylmethylamino-3,4-dihydroxy-acetophenone

A mixture of α-chloro-3,4-dihydroxyacetophenone (18.8, 0.1 mole) and 10 g of potassium iodide in 240 ml of dimethylformamide was stirred at room temperature for 15 minutes, then 13.0 g of benzylmethylamine in 20 ml of dimethylformamide was added slowly. Stirring was continued for 16 hours. The mixture was poured on ice-water and extracted with 3 portions (100 ml each) of ether. The combined ether layers were extracted with 2 portions (100 ml each) of 4N hydrochloric acid. The acid layer was basified with 5% sodium hydroxide solution and extracted with 3 portions of ether (100 ml each). The ether layers were combined, dried over magnesium sulfate and used in part B.

B. 1-Benzylmethylamino-2-(3,4-dihydroxyphenyl)-2-butanol

The ether solution from Part A was added dropwise to an ether solution of 0.4 mole of ethylmagnesium bromide and the mixture stirred at room temperature for 4 hours. The mixture was decomposed with saturated ammonium chloride solution and the ether phase dried over magnesium sulfate and concentrated to a gum which was used in Part C.

C. 1-Benzylmethylamino-2[3,4-bis(3,3dimethylbutanoyloxy) phenyl]-2-butanol

The gummy residue from Part B in 200 ml of dry triethylamine was mixed with 32.4 g (0.24 mole) of 3,3-dimethylbutanoyl chloride and the mixture was stirred at room temperature for 2 days, then concentrated to a gum. This was taken up in methylene chloride solution and passed through a silica gel HPLC column whereby a small amount of triester byproduct was separated in the early fractions. The later fractions combined the product in pure form, and were combined and concentrated to a gum.

D. 2-[3,4-Bis(3,3-dimethylbutanoyloxy)phenyl]-1-methylamino-2-butanol hydrochloride

In the same manner as described in Example III D, the product from Part C was hydrogenated to remove the protecting benzyl group and the resulting base was converted to its crystalline hydrochloride salt.

By following the procedures of examples I to VI but using acids other than hydrochloric, different acid addition salts may be made. Acids suitable for such use include for example sulfuric, phosphoric, benzoic,

mandelic, nicotinic, malic, malonic, boric, succinic, lactic, citric, maleic, ethanesulfonic or fumaric acid.

Examples VII to XV illustrate suitable solutions for topical use. The numerical values are in weight-percent.

### Example VII

| | |
|---|---|
| 3,3-Dimethyl-butanoic acid diester of epinephrine hydrochloride | 0.1 |
| Sodium chloride | 0.85 |
| Edetate disodium | 0.02 |
| Phosphate buffer (to pH 6.0) | 0.1 |
| Benzalkonium chloride | 0.005 |
| Water | q.s.100 |

### Example VIII

| | |
|---|---|
| 3,3-Dimethyl-butanoic acid diester of α-methyl-epinephrine hydrochloride | 0.1 |
| Sodium chloride | 0.85 |
| Edetate disodium | 0.02 |
| Hydroxyethylcellulose | 0.3 |
| Polyvinyl alcohol | 0.1 |
| Phosphate buffer (to pH 6.0) | 0.1 |
| Benzalkonium chloride | 0.005 |
| Water | q.s. 100 |

### Example IX

| | |
|---|---|
| 3,3-Dimethyl-pentanoic acid diester of α-methyl epinephrine perchlorate | 0.1 |
| Sodium chloride | 0.85 |
| Edetate disodium | 0.02 |
| Phosphate buffer (to pH 6.0) | 0.1 |
| Thimerosal | 0.004 |
| Water | q.s. 100 |

### Example X

Same as EXAMPLE II except that thimerosal (0.004) was substituted for benzalkonium chloride (0.005).

### Examples XI—XIV

Same as EXAMPLES I to IV respectively, except that citrate buffer (to pH 6.0) substituted for phosphate buffer.

Example XV

| | |
|---|---|
| 2,3,3-Trimethyl-butanoic acid diester of epinephrine ethanesulfonate | 0.05 |
| Sodium chloride | 0.85 |
| Edetate sodium | 0.02 |
| Acetate buffer (to pH 6.0) | 0.1 |
| Benzalkonium chloride | 0.005 |
| Water | q.s. 100 |

Pharmaceutical compositions comprise an effective amount of a compound of the present invention as active ingredient together with pharmaceutically acceptable carriers and/or diluents.

The concentration of the active ingredient may be varied over a range of about 0.001 to 0.2%.

In addition to having the considerably higher activity and longer duration of activity as compared with the known compounds discussed above, the compounds of the present invention produce little or no initial increase in intraocular pressure upon administration, and where such increase is produced, its duration is much shorter than that produced by the dipivalyl ester.

The compounds of the present invention also produce a lower contralateral effect and mydriasis as compared to the pivalyl ester.

**Claims**

1. Compounds of formula I

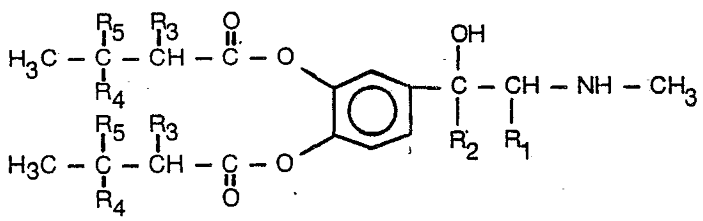

(I)

wherein

each of $R_1$, $R_2$ and $R_3$ is independently hydrogen, methyl or ethyl, and

each of $R_4$ and $R_5$ is independently methyl or ethyl

and their nontoxic, pharmaceutically acceptable acid addition salts.

2. Compounds according to claim 1 wherein the acid addition salt is the hydrochloride.

3. Compounds according to claim 1 wherein the acid addition salt is the perchlorate.

4. Compounds according to claim 1 wherein $R_3$ is hydrogen.

5. Compounds according to claim 4 wherein $R_4$ and $R_5$ are methyl.

6. Compounds according to claim 5 wherein $R_2$ is hydrogen.

7. Compounds according to claim 6 wherein $R_1$ is hydrogen.

8. Compounds according to claim 6 wherein $R_1$ is methyl.

9. A compound according to claim 1 wherein $R_1$, $R_3$, $R_4$ and $R_5$ are methyl and $R_2$ is hydrogen.

10. A compound according to claim 1 wherein $R_1$, $R_2$ and $R_3$ are hydrogen, $R_4$ is methyl and $R_5$ is ethyl.

11. A compound according to claim 1 wherein $R_1$, $R_2$, $R_4$ and $R_5$ are methyl and $R_3$ is hydrogen.

12. A process for the preparation of compounds according to any of claims 1 to 11 which comprises reducing a compound of formula II;

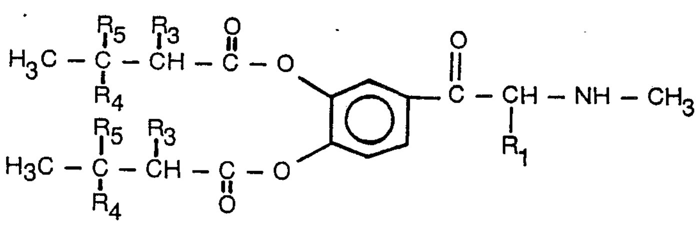

(II)

or reacting a compound of formula II with methyl or ethyl magnesium halide or an equivalent or acylating a compound of formula III

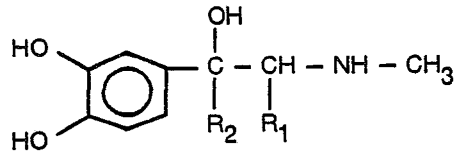

(III)

with an acylating derivative of an acid of formula IV;

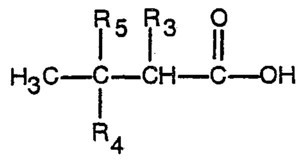

(IV)

and optionally connecting said products to a pharmaceutically acceptable acid salt.

13. A pharmaceutical composition comprising an effective amount of a compound according to any of claims 1 to 11 and pharmaceutical acceptable carriers and/or diluents.

**Revendications**

1. Composés de formule I

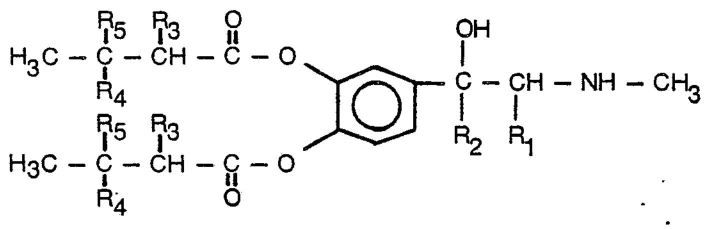

(I)

formule dans laquelle:

— chacun parmi $R_1$, $R_2$ et $R_3$ est, indépendamment, un atome d'hydrogène, un groupe méthyle ou un groupe éthyle, et

— chacun parmi $R_4$ et $R_5$ est, indépendamment, un groupe méthyle ou un groupe éthyle,

et leurs sels d'addition acide non toxiques, pharmaceutiquement acceptables.

2. Composés selon la revendication 1, dans lesquels le sel d'addition acide est le chlorhydrate.

3. Composés selon la revendication 1, dans lesquels le sel d'addition acide est le perchlorate.

4. Composés selon la revendication 1, dans lesquels $R_3$ est un atome d'hydrogène.

5. Composés selon la revendication 4, dans lesquels $R_4$ et $R_5$ sont des groupes méthyle.

6. Composés selon la revendication 5, dans lesquels $R_2$ est un atome d'hydrogène.

7. Composés selon la revendication 6, dans lesquels $R_1$ est un atome d'hydrogène.

8. Composés selon la revendication 6, dans lesquels $R_1$ est un groupe méthyle.

9. Composé selon la revendication 1, dans lequel $R_1$, $R_3$, $R_4$ et $R_5$ sont des groupes méthyle et $R_2$ est un atome d'hydrogène.

10. Composé selon la revendication 1, dans lequel $R_1$, $R_2$ et $R_3$ sont des atomes d'hydrogène, $R_4$ est un groupe méthyle et $R_5$ est un groupe éthyle.

11. Composé selon la revendication 1, dans lequel $R_1$, $R_2$, $R_4$ et $R_5$ sont des groupes méthyle et $R_3$ est un atome d'hydrogène.

12. Procédé pour la préparation de composé selon l'une des revendications 1 à 11, qui consiste à réduire un composé de formule II:

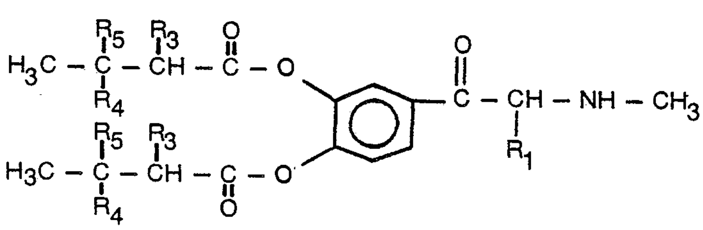

(II)

ou à faire réagir un composé de formule II avec un halogénure de méthyl ou éthyl magnésium ou un équivalent ou à réaliser l'acylation d'un composé de formule III

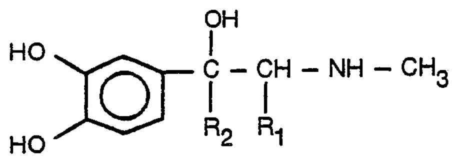

(III)

avec un dérivé acylant d'un acide de formule IV:

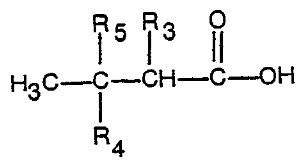

(IV)

et facultativement à combiner lesdits produits avec un sel d'acide pharmaceutiquement acceptable.

13. Composition pharmaceutique comprenant une quantité efficace d'un composé selon l'une des revendications 1 à 11 et des véhicules et/ou diluants pharmaceutiquement acceptables.

**Patentansprüche**

1. Verbindungen der Formel I

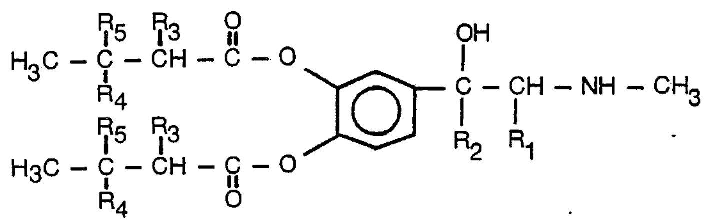

(I)

worin jedes

$R_1$, $R_2$ und $R_3$, unabhängig voneinander, Wasserstoff, Methyl oder Ethyl ist, und

jedes $R_4$ und $R_5$, unabhängig voneinander, Methyl oder Ethyl ist und

ihre nichttoxischen, pharmazeutisch annehmbaren Säureadditionssalze.

2. Verbindungen nach Anspruch 1, worin das Säureadditionssalz das Hydrochlorid ist.

3. Verbindungen nach Anspruch 1, worin das Säureadditionssalz das Perchlorat ist.

4. Verbindungen nach Anspruch 1, worin $R_3$ Wasserstoff ist.

5. Verbindungen nach Anspruch 4, worin $R_4$ und $R_5$ Methyl sind.

6. Verbindungen nach Anspruch 5, worin $R_2$ Wasserstoff ist.

7. Verbindungen nach Anspruch 6, worin $R_1$ Wasserstoff ist.

8. Verbindungen nach Anspruch 6, worin $R_1$ Methyl ist.

9. Verbindung nach Anspruch 1, worin $R_1$, $R_3$, $R_4$ und $R_5$ Methyl sind und $R_2$ Wasserstoff ist.

10. Verbindung nach Anspruch 1, worin $R_1$, $R_2$ und $R_3$ Wasserstoff sind, $R_4$ Methyl ist und $R_5$ Ethyl ist.

11. Verbindung nach Anspruch 1, worin $R_1$, $R_2$, $R_4$ und $R_5$ Methyl sind und $R_3$ Wasserstoff ist.

12. Verfahren zur Herstellung der Verbindungen nach einem der Ansprüche 1 bis 11, welches die Reduktion einer Verbindung der Formel II

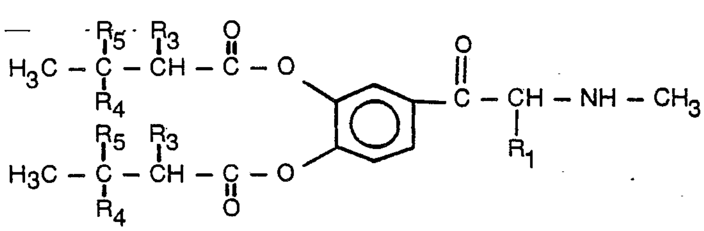

(II)

oder die Umsetzung einer Verbindung der Formel II mit Methyl- oder Ethylmagnesiumhalogenid oder einem Äquivalent oder die Acylierung einer Verbindung der Formel III

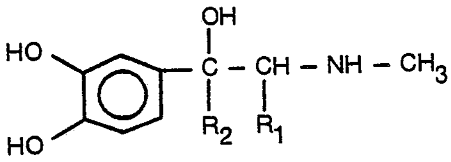

(III)

mit einem acylierenden Derivat einer Säure der Formel IV

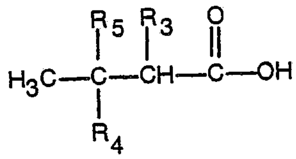

(IV)

und gegebenenfalls die Verbindung der Produkte mit einem pharmazeutisch annehmbaren Säuresalz umfaßt.

13. Pharmazeutische Zusammensetzung, umfassend eine wirksame Menge einer Verbindung nach einem der Ansprüche 1 bis 11 und pharmazeutisch annehmbare Träger und/oder Verdünnungsmittel.